# EUROPÄISCHE PATENTSCHRIFT

(11) **EP 1 845 955 B1**
(45) Veröffentlichungstag und Bekanntmachung des Hinweises auf die Patenterteilung: **27.05.2015**
(21) Anmeldenummer: 06706680.3
(22) Anmeldetag: 06.02.2006
(51) Int. Cl.: A61K 9/20, A61K 31/135

(54) **VERFAHREN ZUR HERSTELLUNG EINER GEGEN MISSBRAUCH GESICHERTEN DARREICHUNGSFORM**
METHOD FOR THE PRODUCTION OF A TAMPER-PROOF FORM OF ADMINISTRATION
PROCEDE DE PRODUCTION D'UNE FORME GALENIQUE PROTEGEE CONTRE UNE UTILISATION ABUSIVE

(30) Priorität: 04.02.2005 DE 102005005449
(43) Veröffentlichungstag der Anmeldung: 24.10.2007
(73) Patentinhaber: Grünenthal GmbH, 52078 Aachen (DE)
(72) Erfinder: ARKENAU-MARIC, Elisabeth, 50931 Köln (DE); BARTHOLOMÄUS, Johannes, 52080 Aachen (DE)
(74) Vertreter: Kutzenberger, Helga
(86) Internationale Anmeldenummer: PCT/EP2006/001025
(87) Internationale Veröffentlichungsnummer: WO 2006/082097

(56) Entgegenhaltungen:
- WO-A-99/44591
- WO-A-2004/026262
- WO-A-2005/016314
- WO-A-2005/102286
- US-A1- 2003 068 392

## Beschreibung

*Die vorliegende Erfindung betrifft ein Verfahren zur Herstellung einer festen Darreichungsform mit zumindest vermindertem Missbrauchspotential, dadurch gekennzeichnet, dass man*
*a) zu einer Formulierungsmischung, enthaltend wenigstens einen Wirkstoff mit Missbrauchspotential (A), wenigstens ein Antioxidans als Hilfsstoff (B) und wenigstens ein synthetisches oder natürliches Polymer (C), das eine Bruchfestigkeit von mindestens 500 N aufweist, ein Lösungsmittel für das Polymer (C) zumindest in solchen Mengen zugibt, dass die Formulierungsmischung gleichmäßig befeuchtet wird;*
*b) gegebenenfalls die so zumindest befeuchtete Masse auf Teilmassen verteilt;*
*c) die Masse(n) trocknet; und*
*d) zu der Darreichungsform formt;*
*wobei Polymer (C) in solchen Mengen eingesetzt wird, dass auch die Darreichungsform eine Mindestbruchfestigkeit von mindestens 500 N aufweist.*

Eine Vielzahl von pharmazeutischen Wirkstoffen weist neben einer ausgezeichneten Wirksamkeit auf ihrem betreffenden Anwendungsgebiet auch ein Missbrauchspotential auf, d.h. sie können von einem Missbraucher eingesetzt werden, um Wirkungen herbeizuführen, die nicht ihrem Bestimmungszweck entsprechen.

So werden beispielsweise Opiate, die eine exzellente Wirksamkeit bei der Bekämpfung von starken bis sehr starken Schmerzen zeigen, von Missbrauchem häufig zum Einleiten rauschartiger, euphorisierender Zustände verwendet.

Um Missbrauch zu ermöglichen, werden die entsprechenden Darreichungsformen wie Tabletten oder Kapseln vom Missbraucher zerkleinert, z. B. gemörsert, der Wirkstoff aus dem so erhaltenen Pulver mit Hilfe einer vorzugsweise wässrigen Flüssigkeit extrahiert und die resultierende Lösung, ggf. nach Filtration durch Watte oder Zellstoff, parenteral, insbesondere intravenös, appliziert. Bei dieser Art der Verabreichung kommt es zu einem gegenüber der oralen, missbräuchlichen Applikation noch zusätzlich beschleunigten Anfluten des Wirkstoffes mit dem vom Mißbraucher gewünschten Ergebnis, nämlich dem Kick. Dieser Kick wird auch erreicht, wenn die gepulverte Darreichungsform nasal appliziert, d. h. geschnupft wird.

Da retardierte, orale Darreichungsformen, die Wirkstoffe mit Mißbrauchspotential enthalten, üblicherweise selbst bei einer oralen Einnahme von missbräuchlich hohen Mengen nicht zu dem vom Mißbraucher gewünschten Kick führen, werden auch diese zum Missbrauch zerkleinert und extrahiert.

Zur Verhinderung des Missbrauchs wurde in dem US-A- 4,070,494 vorgeschlagen, der Darreichungsform ein quellbares Mittel zuzusetzen. Dieses quillt bei der Zugabe von Wasser zur Extraktion des Wirkstoffes auf und bewirkt, dass das vom Gel separierte Filtrat nur eine geringe Menge an Wirkstoff enthält.

Ein entsprechender Ansatz zur Verhinderung des parenteralen Missbrauchs liegt auch der in der WO 95/20947 offenbarten Mehrschichttablette zugrunde, die den Wirkstoff mit Missbrauchspotential und mindestens einen Gelbildner jeweils in unterschiedlichen Schichten getrennt aufweist.

Ein weiterer Ansatz zur Verhinderung des parenteralen Missbrauchs wird in der WO 03/015531 A2 offenbart. Dort wird eine Darreichungsform enthaltend ein analgetisches Opioid und einen Farbstoff als aversives Mittel beschrieben. Die Farbe, die durch unzulässige Manipulation der Darreichungsform freigesetzt wird, soll den Mißbraucher davon abhalten, diese manipulierte Darreichungsform zu verwenden.

WO 2005/102286 *betrifft ein Verfahren zur Herstellung einer gegen Missbrauch gesicherten, festen Darreichungsform enthaltend wenigstens einen Wirkstoff mit Missbrauchspotential und ein Bindemittel mit einer Bruchfestigkeit von ≥500 N, indem man auf eine Mischung umfassend den Wirkstoff und das Bindemittel Ultraschall und eine Kraft einwirken lässt.*

Eine weitere bekannte Möglichkeit zur Erschwerung des Missbrauchs besteht darin, der Darreichungsform Antagonisten der Wirkstoffe, wie z. B. Naloxon oder Naltexon im Fall von Opioiden, oder Verbindungen, die zu physiologischen Abwehrreaktionen führen, wie z. B. Raolix Ipecacuama = Brechwurz, der Darreichungsform zuzusetzen.

Da aber nach wie vor in den meisten Fällen für den Missbrauch eine Pulverisierung der Darreichungsform notwendig ist, war es Aufgabe der vorliegenden Erfindung, ein Verfahren zur Herstellung von Darreichungsformen enthaltend Wirkstoffe mit Missbrauchspotential zur Verfügung zu stellen, die bei bestimmungsgemäßer Applikation die gewünschte, vorzugsweise therapeutische Wirkung gewährleisten, aus denen aber die Wirkstoffe nicht durch einfaches Pulverisieren in eine zum Missbrauch geeignete Form übergeführt werden können.

Diese Aufgabe wurde gelöst durch die Bereitstellung des erfindungsgemäßen Verfahren zur Herstellung einer festen Darreichungsform mit zumindest vermindertem Missbrauchspotential dadurch gekennzeichnet, dass man
*a) zu einer Formulierungsmischung, enthaltend wenigstens einen Wirkstoff mit Missbrauchspotential (A), wenigstens ein Antioxidans als Hilfsstoff (B) und wenigstens ein synthetisches oder natürliches Polymer (C), das eine Bruchfestigkeit von mindestens 500 N aufweist, ein Lösungsmittel für das Polymer (C) zumindest in solchen Mengen zugibt, dass die Formulierungsmischung gleichmäßig befeuchtet wird;*
*b) gegebenenfalls die so zumindest befeuchtete Masse auf Teilmassen verteilt;*
*c) die Masse(n) trocknet; und*
*d) zu der Darreichungsform formt;*
*wobei Polymer (C) in solchen Mengen eingesetzt wird, dass auch die Darreichungsform eine Mindestbruchfestigkeit von mindestens 500 N aufweist.*

Durch den Einsatz von Polymeren mit der angegeoenen Mindestbruchfestigkeit (gemessen, wie in der Anmeldung angegeben), in solchen Mengen, dass auch die Darreichungsform eine solche Mindestbruchfestigkeit von mindestens 500 N, vorzugsweise mindestens 1000 N, aufweist, gelingt es, ein Pulverisieren der Darreichungsform mit üblichen Mitteln zu verhindern und damit den anschließenden Missbrauch erheblich zu erschweren bzw. zu unterbinden.

Ohne ausreichende Zerkleinerung ist nämlich eine parenteral, insbesondere intravenöse, gefahrlose Applikation nicht möglich oder die Extraktion des Wirkstoffes daraus dauert für den Missbraucher zu lange bzw. ein Kick bei missbräuchlicher, oralen Einnahme erfolgt nicht, da keine spontane Freisetzung passiert.

Unter einer Zerkleinerung wird erfindungsgemäß die Pulverisierung der Darreichungsform mit üblichen Mitteln, die einem Missbraucher üblicherweise zur Verfügung stehen, wie z. B. ein Mörser und Pistill, ein Hammer, ein Schlegel oder andere gebräuchliche Mittel zum Pulverisieren unter Krafteinwirkung verstanden, wobei ein gegebenenfalls anfallender Feinanteil (Teilchengröße gleich oder kleiner 0,3 mm) von 5 Gew.-% nicht überschritten werden darf.

Die erfindungsgemäß hergestellte Darreichungsform kann auch nicht bei tiefen Temperaturen, beispielsweise unterhalb von -25°C, -40°C oder sogar in flüssigem Stickstoff, mit diesen Methoden zerkleinert werden.

Die erfindungsgemäß hergestellte Darreichungsform, vorzugsweise eine pharmazeutische Darreichungsform, ist daher zur Verhinderung des parenteralen, nasalen und/oder oralen Missbrauchs von Wirkstoffen, vorzugsweise von pharmazeutischen Wirkstoffen, mit Missbrauchspotential geeignet.

Wirkstoffe, vorzugsweise pharmazeutische Wirkstoffe mit Missbrauchspotential sind dem Fachmann ebenso wie deren einzusetzende Mengen und Verfahren zu deren Herstellung bekannt und können als solche, in Form ihrer dementsprechenden Derivate, insbesondere Ester, Ether oder Amide oder jeweils in Form entsprechender physiologisch verträglicher Verbindungen, insbesondere in Form ihrer entsprechenden Salze oder Solvate, als Racemate oder Stereoisomere in der erfindungsgemäß hergestellten Darreichungsform vorliegen. Die erfindungsgemäß hergestellte Darreichungsform kann mehrere pharmazeutische Wirkstoffe enthalten. Vorzugsweise enthält die erfindungsgemäß hergestellte Darreichungsform nur einen bestimmten Wirkstoff.

Die erfindungsgemäße Darreichungsform eignet sich insbesondere zur Verhinderung des Missbrauchs wenigstens eines pharmazeutischen Wirkstoffs, der ausgewählt ist aus der Gruppe umfassend Opioide, Tranquillantien, vorzugsweise Benzodiazepine, Barbiturate, Stimulantien und weitere Betäubungsmittel.

Ganz besonders eignet sich die erfindungsgemäße Darreichungsform zur Verhinderung des Mißbrauchs eines Opioids, Tranquillanz oder eines anderen Betäubungsmittels, das ausgewählt ist aus der Gruppe umfassend N-{1-[2-(4-Ethyl-5-oxo-2-tetrazolin-1-yl)ethyl]-4-methoxymethyl-4-piperidyl}propionanilid (Alfentanil), 5,5-Diallylbarbitursäure (Allobarbital), Allylprodin, Alphaprodin, 8-Chlor-1-methyl-6-phenyl-4H-[1,2,4]triazolo[4,3-a][1,4]-benzodiazepin (Alprazolam), 2-Diethylaminopropiophenon (Amfepramon), (±)-α-Methylphenethylamin (Amfetamin), 2-(α-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3H)-on (Bromazepam), 2-Brom-4-(2-chlorphenyl)-9-methyl-6*H*-thieno[3,2-f][1,2,4]triazolo[4,3-a][1,4]diazepin (Brotizolam), 17-Cyclopropylmethyl-4,5α-epopxy-7α[(S)-1-hydroxy-1,2,2-trimethyl-propyl]-6-methoxy-6,14-endo-ethanomorphinan-3-ol (Buprenorphin), 5-Butyl-5-ethylbarbitursäure (Butobarbital), Butorphanol, (7-Chlor-1,3-dihydro-l-methyl-2-oxo-5-phenyl-2H-1,4-benzodiazepin-3-yl)-dimethyl-carbamat (Camazepam), (1 S,2S)-2-Amino-1-phenyl-1-propanol (Cathin / D-Norpseudoephedrin), 7-Chlor-N-methyl-5-phenyl-3H-1,4-benzodiazepin-2-ylamin-4-oxid (Chlordiazepoxid), 7-Clor-1-methyl-5-phenyl-1 H-1,5-benzodiazepin-2,4(3H,5H)-. dion (Clobazam), 5-(2-Chlorphenyl)-7-nitro-1H-1,4-benzodiazepin-2(3H)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H-*thieno[2,3-e][1,4]diazepin-2(3*H*)-on (Clotiazepam), 10-Chlor-11b-(2chlorphenyl)-2,3,7,11 b-tetrahydrooxazolo[3,2-*d*][1,4]benzodiazepin-6(5*H*)-on (Cloxazolam), (-)-Methyl-[3β-benzoyloxy-2β(1αH,5α*H*)-tropancarboxylat] (Cocain), 4,5α-Epoxy-3-methoxy-17-methyl-7-morphinen-6α-ol (Codein), 5-(1-Cyclohexenyl)-5-ethylbarbitursäure (Cyclobarbital), Cyclorphan, Cyprenorphin, 7-Chlor-5-(2-chlorphenyl)-1*H*-1,4-benzodiazepin-2(3*H*)-on (Delorazepam), Desomorphin, Dextromoramid, (+)-(1-Benzyl-3-dimethylamino-2-methyl-1-phenylpropyl)propionat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6a*R*,10a*R*)-6,6,9-Trimethyl-3-pentyl-6a,7,8,10a-tetrahydro-6*H*-benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethylloflazepat), 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-*endo*-etheno-morphinan-3-ol (Etorphin), *N-*Ethyl-3-phenyl-8,9,10-trinorbornan-2-ylamin (Fencamfamin), 7-[2-(α-Methylphenethylamino)ethyl]-theophyllin) (Fenetyllin), 3-(α-Methylphenethylamino)propionitril (Fenproporex), *N-*(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1*H*-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1 *H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3*S*,6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 6-(2-Chlorphenyl)-2-(4-methyl-1-piperazinylmethylen)-8-nitro-2*H-*imidazo[1,2-a][1,4] benzodiazepin-1(4*H*)-on (Loprazolam), 7-Chlor-5-(2-chlorphenyl)-3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H*-imidazo[2,1-a]isoindol-5-ol (Mazindol), 7-Chlor-2,3-dihydro-1-methyl-5-phenyl-1*H*-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-o-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-*a*][1,4]benzodiazepin (Midazolam), 2-(Benzhydrylsulfinyl)acetamid (Modafinil), 4,5α-Epoxy-17-methyl-7-morphinen-3,6α-diol (Morphin), Myrophin, (±)-*trans-*3-(1,1-Dimethylheptyl)-7,8,10,10α-tetrahydro-1-hydroxy-6,6-dimethyl-6*H*-dibenzo [b, d]pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*)-on (Oxazepam), (*cis-trans*)-10-Chlor-2,3,7,11b-tetrahydro-2-methyl-11b-phenyloxazolo[3,2-*d*][1,4]benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pernolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-phenyl-1 *H*-1,4-benzodiazepin-2(3*H*)-on (Prazepam), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), 5-seo-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3*H*)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1 R,2R)-3-(3-Dimethylamino-1-ethyl-2-methyl-propyl)-phenol, (1 R, 2R, 4S)-2-[Dimethylamino)methyl-4-(p-fluorbenzyloxy)-1-(m-methoxyphenyl)cyclohexanol, (1 R, 2R)-3-(2-Dimethylaminomethyl-cyclohexyl)-phenol, (1S, 2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1 RS, 3RS, 6RS)-6-Dimethylaminomethyl-1-(3-methoxy-phenyl)-cyclohexan-1,3-diol, vorzugsweise als Racemat, 3-(2-Dimethylaminomethyl-1-hydroxy-cyclohexyl)-phenyl 2-(4-isobutyl-phenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt Hydrochloride.

Die erfindungsgemäß hergestellten Darreichungsformen eignen sich insbesondere zur Verhinderung des Missbrauchs eines opioiden Wirkstoffes ausgewählt aus der Gruppe umfassend Oxycodon, Hydromorphon, Morphin, Tramadol und deren physiologisch verträgliche Derivate oder Verbindungen, vorzugsweise deren Salze und Solvate, vorzugsweise deren Hydrochloride.

Weiterhin eignen sich die erfindungsgemäß hergestellten Darreichungsformen insbesondere zur Verhinderung des Missbrauchs eines opioiden Wirkstoffes ausgewählt aus der Gruppe umfassend (1 R, 2R)-3-(3-Dimethylamino-1-ethyl-2-methyl-propyl)-phenol, (2R, 3R)-1-Dimethylamino-3-(3-methoxy-phenyl)-2-methyl-pentan-3-ol, (1RS, 3RS, 6RS)-6-Dimethylaminomethyl-1-(3-methoxy-phenyl)-cyclohexane-1,3-diol, (1 R, 2R)-3-(2-Dimethylaminonethyl-cyclohexyl)-phenol, deren physiologisch verträglichen Salze, vorzugsweise Hydrochloride, physiologisch verträgliche Enantiomere, Stereoisomere, Diastereomere und Racemate und deren physiologisch verträglichen Derivate, vorzugsweise Ether, Ester oder Amide.

Diese Verbindungen bzw. deren Herstellungsverfahren sind in der EP-A-693475 bzw. EP-A-780369 beschrieben.

Zur Erzielung der notwendigen Bruchfestigkeit wird In dem erfindungsgemäßen Verfahren mindestens ein synthetisches oder natürliches Polymer (C) mit einer Bruchfestigkeit, gemessen nach der in der vorliegenden Anmeldung offenbarten Methode, von mindestens 500 N eingesetzt. Bevorzugt wird hierfür mindestens ein Polymeres ausgewählt aus der Gruppe umfassend Polyalkylenoxide, vorzugsweise Polymethylenoxid, Polyethylenoxid, Polypropylenoxid; Polyethylen, Polypropylen, Polyvinylchlorid, Polycarbonat, Polystyrol, Polyacrylat, deren Copolymerisate und Mischungen aus mindestens zwei der genannten Polymeren eingesetzt. Bevorzugt sind hochmolekulare, thermoplastische Polyalkylenoxide. Besonders bevorzugt sind hochmolekulare Polyethylenoxide mit einem Molekulargewicht von mindestens 0,5 Mio., vorzugsweise mindestens 1 Mio. bis 15 Mio., bestimmt durch rheologische Messungen. Diese Polymeren weisen eine Viskosität bei 25 °C von 4500 bis 17600 cP, gemessen an einer 5 Gew.% wässrigen Lösung mit Hilfe eines Brookfield Viskosimeter, Model RVF (Spindel Nr. 2 / Rotationsgeschwindigkeit 2 rpm), von 400 bis 4000 cP, gemessen an einer 2 Gew.% wässrigen Lösung mit Hilfe des genannten Viskosimeters (Spindel Nr. 1 bzw. 3 / Rotationsgeschwindigkeit 10 rpm) bzw. von 1650 bis 10000 cP, gemessen an einer 1 Gew.% wässrigen Lösung mit Hilfe des genannten Viskosimeters (Spindel Nr. 2 /Rotationsgeschwindigkeit 2 rpm) auf.

Die Polymeren werden bevorzugt als Pulver eingesetzt. Sie sollen in Wasser löslich sein.

Des weiteren können zusätzlich zur Erzielung der notwendigen Bruchfestigkeit bei den erfindungsgemäßen Verfahren mindestens ein natürliches oder synthetisches Wachs (D) mit einer Bruchfestigkeit, gemessen nach der in der vorliegenden Anmeldung offenbarten Methode, von mindestens 500 N eingesetzt werden. Bevorzugt sind Wachse mit einem Erweichungspunkt von mindestens 60°C. Besonders bevorzugt sind Carnaubawachs und Bienenwachs. Ganz besonders bevorzugt ist Carnaubawachs. Carnaubawachs ist ein natürliches Wachs, das aus den Blättern der Carnaubapalme gewonnen wird und einen Erweichungspunkt von wenigstens 80 °C aufweist. Beim zusätzlichen Einsatz der Wachskomponente wird diese zusammen mit wenigstens einem Polymeren (C) in solchen Mengen eingesetzt, dass die erfindungsgemäß hergestellte Darreichungsform eine Bruchfestigkeit von mindestens 500 N aufweist.

Vorzugsweise wird die Komponente (C) in einer Menge von 20 bis 99,9 Gew.%, besonders bevorzugt von wenigstens 30 Gew.%, ganz besonders bevorzugt von wenigstens 40 Gew.%, bezogen auf das Gesamtgewicht der Darreichungsform, eingesetzt.

Als Hilfsstoffe (B) können die üblichen für die Formulierung von festen Darreichungsformen bekannten Hilfsstoffe verwendet werden.

*Erfindungsgemäß sind dies Antioxidantien. Vorzugsweise sind dies Weichmacher, wie Triacetin und Polyethylenglykol, Hilfsstoffe, welche die Wirkstofffreisetzung beeinflussen, vorzugsweise hydrophobe oder hydrophile, vorzugsweise hydrophile Polymere, ganz besonders bevorzugt Hydroxypropylmethylcellulose oder Hydroxypropylcellulose."*

Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt. Als Antioxidantien eignen sich Ascorbinsäure, Butylhydroxyanisol, Butylhydroxytoluol, Salze der Ascorbinsäure, Monothioglyzerin, phosphorige Säure, Vitamin C, Vitamin E und dessen Derivate, Natriumbisulfit, besonders bevorzugt Butylhydroxytoluol (BHT) oder Butylhydroxyanisol (BHA) und α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Zur Durchführung des erfindungsgemäßen Verfahrens wird wenigstens ein Wirkstoff mit Missbrauchspotential (A), wenigstens ein Polymeres (C) und gegebenenfalls ein Wachs (D), gegebenenfalls mindestens eine der nachfolgend aufgeführten gegebenenfalls vorhandenen weiteren missbrauchsverhindemden Komponenten (a) bis (f), *wenigstens ein Antioxidans als Hilfsstoff (B) und gegebenenfalls weitere Hilfsstoffe (B) wie Weichmacher und*/*oder retardierende Hilfsstoffe* unter Zugabe eines Lösungsmittels für das Polymere (C) zu der Darreichungsform verarbeitet.

Dazu werden die Komponenten (A), (B), (C) und die ggf. vorhandene Komponente (D) sowie ggf. mindestens eine der ggf. vorhandenen weiteren missbrauchsverhindernden Komponenten (a) bis (f) gemischt oder, wenn notwendig separat unter Zugabe der Komponente (C) und gegebenenfalls der Komponente (D) gemischt und die resultierende Formulierungsmischung bzw. die resultierenden Formulierungsmischungen nach Zugabe des Lösungsmittels und ggf. nach einer Granulierung zu der Darreichungsform geformt.

Die Mischung der Komponenten (A), (B), (C) und ggf. (D) sowie bzw. der ggf. vorhandenen weiteren Komponenten (a) bis (f) mit der Komponenten (C) und der ggf. vorhandenen Komponente (D) erfolgt ggf. jeweils in einem dem Fachmann bekannten Mischgerät. Das Mischgerät kann beispielsweise ein Wälzmischer, Schüttelmischer, Schermischer oder Zwangsmischer sein.

Die Zugabe des Lösungsmittels für das Polymere (C) erfolgt zumindest in solchen Mengen, das die Formullerungsmischung gleichmäßig befeuchtet wird.

Als Lösungsmittel für das Polymere (C) eignen sich vorzugsweise wässrige Lösungsmittel, wie Wasser, Mischungen von Wasser und aliphatischen Alkoholen, vorzugsweise Alkoholen mit C₁ bis C₆, Estern, Ethern, Kohlenwasserstoffen, besonders bevorzugt destilliertes Wasser, allein oder in Mischung mit kurzkettigen Alkoholen, wie Methanol, Ethanol, Isopropanol, Butanol zu wässrigen Alkohollösungen.

Die Zugabe des Lösungsmittels erfolgt vorzugsweise durch Rühren. Anschließend wird die gleichmäßig befeuchtete Masse getrocknet. Die Trocknung erfolgt vorzugsweise unter Wärmeeinwirkung bei Temperaturen, bei denen eine Verfärbung der Masse ausgeschlossen werden kann. Durch einfache Vorversuchte ist diese Temperatur feststellbar.

Vor oder nach der Trocknung kann die Masse auf Teilmassen aufgeteilt werden, die vorzugsweise jeweils der Masse einer Einheit der Darreichungsform entsprechen. Die entsprechend getrockneten Massen werden dann zu der Darreichungsform geformt.

Vorzugsweise geschieht dies unter Einsatz von Tablettenpressen.

Es ist auch möglich, die Befeuchtung der Formulierungsmischung so durchzuführen, dass vor der Zugabe des Lösungsmittels die Formulierungsmischung, vorzugsweise in Formen auf Teilmassen verteilt, in einem flüssigen Dispergierungsmittel unter Rühren zu dispergieren und dann das Lösungsmittel zuzugeben. Die Polymerkomponente (C) ist in dem Dispergiermittel nicht löslich, das mit dem Lösungsmittel mischbar sein muss.

Als Dispergiermittel eignen sich vorzugsweise hydrophile Lösungsmittel, wie aliphatische Alkohole, Ketone, Ester. Kurzkettige Alkohole werden bevorzugt eingesetzt.

Alternativ kann die Befeuchtung der Formulierungsmischung auch so erfolgen, dass das Lösungsmittel als Schaum in die Formulierungsmischung eingearbeitet werden kann. Vorzugsweise wird ein solcher Schaum des Lösungsmittels mit Hilfe hochtouriger Mixer, vorzugsweise unter Zugabe üblicher Schaumstabilisatoren, hergestellt. Beispielsweise eignen sich als Stabilisatoren hydrophile Polymere wie z. B. Hydroxypropylmethylcellulose.

Vorzugsweise wird auch der Schaum unter Rühren in die Formulierungsmischung eingearbeitet, wodurch vorzugsweise eine granulierte Masse erhalten wird.

Die granulierte Masse wird vor oder nach ihrer Aufteilung in Teilmassen, die vorzugsweise der Masse einer Einheit der Darreichungsform entspricht, getrocknet und anschließend zur Darreichungsform geformt.

Die Trocknung und Formung kann vorzugsweise wie vorstehend angegeben erfolgen.

Das erfindungsgemäße Verfahren kann auch so durchgeführt werden, dass zu der Formulierungsmischung soviel Lösungsmittel zugegeben wird, dass eine formbare Paste entsteht.

Eine solche Paste kann vor oder nach ihrer Trocknung, die wie vorstehend aufgeführt, durchgeführt werden kann, in Teilmassen aufgeteilt werden und die getrockneten Massen gegebenenfalls nach einer weiteren Verteilung jeweils auf eine Masse entsprechend der Masse einer Einheit der Darreichungsform zur Darreichungsform geformt oder umgeformt werden.

Dabei ist es möglich, die Teilmassen in Form von Strängen auszubilden, die mit Hilfe eines Siebes oder eines Strangformers erzeugt werden können. Die getrockneten Stränge werden vorzugsweise vereinzelt und zur Darreichungsform geformt. Diese Formung erfolgt vorzugsweise mit Hilfe einer Tablettenpresse, unter Einsatz von Formwalzen oder mit Walzen ausgerüsteten Formbändem.

Es ist auch möglich, die Paste zu einem flächenförmigen Gebilde zu verarbeiten und aus dem getrocknete Gebilde die Darreichungsform zu stanzen.

Vorteilhafterweise wird die Paste mit Hilfe eines Extruders verarbeitet, wobei je nach Gestaltung der Extrusionsdüse diese Stränge oder flächenförmige Gebilde erzeugt werden, die durch Abschlagen oder Schneiden bzw. Stanzen vereinzelt werden. Die vereinzelten Teilmassen können, wie vorstehend ausgeführt, zu der Darreichungsform geformt oder verformt werden. Entsprechende Vorrichtungen sind dem Fachmann bekannt.

Dabei kann das erfindungsgemäße Verfahren kontinuierlich oder diskontinuierlich durchgeführt werden.

Es ist auch möglich, zu der Formulierungsmischung soviel Lösungsmittel zuzugeben, dass zumindest die Polymerkomponente (C) gelöst wird. Eine solche Lösung oder Dispersion/Suspension wird vorzugsweise zu einem flächenförmigen Gebilde verarbeitet, wobei bevorzugt ein Extruder mit einer Flachdüse zum Einsatz kommt oder die Lösung auf eine flächenförmige, ebene Unterlage ausgegossen wird.

Nach Trocknung, wie vorstehend angegeben, können aus den flächenförmigen Gebilden die Darreichungsformen durch Stanzen oder Kalandrieren erhalten werden. Es ist auch möglich, die Lösung, wie vorstehend angegeben, zu Strängen zu verarbeiten und diese, bevorzugt nach ihrer Trocknung, zu vereinzeln und zur Darreichungsform zu formen.

Alternativ kann die Lösung auch in solchen Teilmengen aufgeteilt werden, dass sie nach dem Trocknen jeweils der Masse einer Einheit der Darreichungsform entspricht, wobei vorzugsweise dafür bereits Formen entsprechend der Form einer Einheit der Darreichungsform eingesetzt werden.

Sofern die Lösung in beliebige Teilmengen aufgeteilt wird, können die Teilmengen nach dem Trocknen gegebenenfalls wieder vereinigt und zu der Darreichungsform geformt werden, wie z. B. in eine Kapsel abgefüllt oder zu einer Tablette verpresst werden.

Vorzugsweise werden die mit Lösungsmittel versetzten Formulierungsmischungen bei Temperaturen von 20°C bis 40°C verarbeitet, wobei außer bei der Trocknung zur Entfernung des Lösungsmittels und des ggf. vorhandenen Dispergierungsmittels keine höheren Temperaturen angewendet werden. Gegebenenfalls kann nach der Formgebung zur Darreichungsform nochmals eine Trocknung entsprechend der vorstehend beschriebenen Trocknung erfolgen.

Wie bereits ausgeführt, kann die erfindungsgemäß hergestellte Darreichungsform in multipartikulärer Form, bevorzugt in Form von Mikrotabletten, Mikrokapseln, Mikropellets, Granulaten, Sphärolden, Perlen oder Pellets, ggf. in Kapseln abgefüllt oder zu Tabletten verpresst, vorzugsweise zur oralen Verabreichung, vorliegen. Vorzugsweise weisen die multipartikulären Formen eine Größe bzw. Größenverteilung im Bereich von 0,1 bis 3 mm, besonders bevorzugt im Bereich von 0,5 bis 2 mm auf. Je nach gewünschter Darreichungsform *wird wenigstens ein Antioxidans als Hilfsstoff (B) und ggf. weitere übliche Hilfsstoffe (B) zur Formulierung der Darreichungsform mitverwendet.*

Die durch das erfindungsgemäße Verfahren erhaltene Darreichungsformen zeichnen sich dadurch aus, dass sie aufgrund ihrer Härte von mindestens 500 N mit Hilfe von üblichen, einem Missbraucher zur Verfügung stehenden Zerkleinerungsmitteln, wie Mörser und Pistill, nicht zu pulverisieren sind. Ein oraler, parenteraler, insbesondere intravenöser oder nasaler Missbrauch ist dadurch praktisch ausgeschlossen. Um jedoch jeden möglichen Missbrauch der erfindungsgemäß hergestellten Darreichungsformen vorzubeugen, können diese Darreichungsformen in einer bevorzugten Ausführungsform als Hilfsstoffe (B) weitere Missbrauchs-erschwerende bzw. -verhindernde Mittel enthalten.

So kann die erfindungsgemäß hergestellte, gegen Missbrauch gesicherte Darreichungsform, die neben einem oder mehreren Wirkstoffen mit Missbrauchspotential (A), *wenigstens einem Antioxidans als Hilfsstoff (B), mindestens einem härtebildenden Polymer (C) und ggf. mindestens einen Wachs (D) noch wenigstens eine der nachfolgenden Komponenten (a)-(f) als weitere Hilfsstoffe (B) aufweisen:*
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wässrigen Flüssigkeit aus der Darreichungsform gewonnenen Extrakt ein Gel bildet, welches vorzugsweise beim Einbringen in eine weitere Menge einer wässrigen Flüssigkeit visuell unterscheidbar bleibt,
(c) wenigstens einen Antagonisten für jeden der Wirkstoffe mit Missbrauchspotential,
(d) wenigstens ein Emetikum.
(e) wenigstens einen Farbstoff als aversives Mittel
(f) wenigstens einen Bitterstoff

Die Komponenten (a) bis (f) sind jeweils für sich allein zusätzlich zur Sicherung der erfindungsgemäß erhaltenen Darreichungsform gegen Missbrauch geeignet. So eignet sich die Komponente (a) bevorzugt zur Sicherung gegen nasalen, oralen und/oder parenteralen, vorzugsweise intravenösen, Missbrauch, die Komponente (b) bevorzugt gegen parenteralen, besonders bevorzugt intravenösen und/oder nasalen Missbrauch, die Komponente (c) bevorzugt gegen nasalen und/oder parenteralen, besonders bevorzugt intravenösen, Missbrauch, die Komponente (d) vorzugsweise gegen parenteralen, besonders bevorzugt intravenösen, und/oder oralen und/oder nasalen Missbrauch, die Komponente (e) als visuelles Abschreckungsmittel gegen oralen oder parenteralen Missbrauch und die Komponente (f) gegen oralen oder nasalen Missbrauch. Durch die erfindungsgemäße Mitverwendung von wenigstens einer der vorstehend genannten Komponenten, gelingt es, bei den durch das erfindungsgemäße Verfahren erhaltenen Darreichungsformen noch effektiver den Missbrauch vorzubeugen.

Beispielsweise kann die erfindungsgemäß erhaltene Darreichungsform auch zwei oder mehrere der Komponenten (a)-(f) in einer Kombination aufweisen, vorzugsweise (a), (b) und ggf. (c) und/oder (f) und/oder (e) bzw. (a), (b) und ggf. (d) und/oder (f) und/oder (e).

In einer weiteren Ausführungsform kann die erfindungsgemäß erhaltene Darreichungsform sämtliche Komponenten (a)-(f) aufweisen.

Sofern die erfindungsgemäß erhaltene Darreichungsform eine Missbrauchsverhindernde Komponente (a) umfasst, kommen als den Nasen- und/oder Rachenraum reizende Stoffe erfindungsgemäß sämtliche Stoffe in Betracht, die bei entsprechender Applikation Ober den Nasen- und/oder Rachenraum eine Reaktion des Körpers hervorrufen, die entweder für den Missbraucher so unangenehm ist, dass er die Applikation nicht weiter fortsetzen will oder kann, z.B. ein Brennen, oder die auf physiologische Art und Weise einer Aufnahme des entsprechenden Wirkstoffes entgegenwirken, z.B. über eine vermehrte nasale Sekretbildung oder Niesen. Diese üblicherweise den Nasen- und/oder Rachenraum reizenden Stoffe können auch bei parenteraler, insbesondere intravenöser, Applikation ein sehr unangenehmes Gefühl bis hin zu unerträglichen Schmerzen verursachen, so dass der Missbraucher die Einnahme nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff., beschrieben.

Unter Darreichungseinheit wird eine separate bzw. separierbare Dosiseinheit, wie z. B. eine Tablette oder eine Kapsel, verstanden.

Vorzugsweise kann der erfindungsgemäßen Darreichungsform als Komponente (a) einer oder mehrere Inhaltsstoffe wenigstens einer Scharfstoffdroge, ausgewählt aus der Gruppe bestehend aus Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe bestehend aus Capsici Fructus (Paprika), Capsici Fructus acer (Cayennepfeffer) und Piperis nigri Fructus (Pfeffer), hinzugefügt werden.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist wenigstens ein Inhaltsstoff der Scharfstoffdrogen ausgewählt aus der Gruppe bestehend aus Myristicin, Elemicin, Isoeugenol, α-Asaron, Safrol, Gingerolen, Xanthorrhizol, Capsaicinoiden, vorzugsweise Capsaicin, Capsaicin- Derivate, wie N-vanillyl -9E-octadecenamid, Dihydrocapsaicin, Nordihydrocapsaicin, Homocapsaicin, Norcapsaicin, und Nomorcapsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und von diesen Inhaltsstoffen abgeleiteten Verbindungen.

Vorzugsweise kann die erfindungsgemäß erhaltene Darreichungsform die Pflanzenteile der entsprechenden Scharfstoffdrogen in einer Menge von 0,01 bis 30 Gew.-%, besonders bevorzugt 0,1 bis 0,5 Gew.-%, jeweils bezogen auf das Gesamtgewicht der Darreichungseinheit, enthalten.

Kommen ein oder mehrere Inhaltsstoffe entsprechender Scharfstoffdrogen zum Einsatz, beträgt deren Menge in einer erfindungsgemäß erhaltenen Darreichungseinheit bevorzugt 0,001 bis 0,005 Gew.-%, bezogen auf das Gesamtgewicht der Darreichungseinheit.

Eine weitere Möglichkeit bei der erfindungsgemäß erhaltenen Darreichungsform gegen Missbrauch vorzubeugen, besteht darin, wenigstens ein viskositätserhöhendes Mittel als weitere Missbrauchs-verhindemde Komponente (b) der Darreichungsform zuzusetzen, das in einem mit Hilfe einer notwendigen Mindestmenge an einer wässrigen Flüssigkeit, vorzugsweise als ein aus der Darreichungsform gewonnenes wässriges Extrakt, ein Gel bildet, das kaum gefahrlos applizierbar ist und vorzugsweise beim Einbringen in eine weitere Menge einer wässrigen Flüssigkeit visuell unterscheidbar bleibt.

Visuelle Unterscheidbarkeit im Sinne der vorliegenden Erfindung bedeutet, dass das mit Hilfe einer notwendigen Mindestmenge an wässriger Flüssigkeit gebildete, Wirkstoff-haltige Gel beim Einbringen vorzugsweise mit Hilfe einer Injektionsnadel, in eine weitere Menge wässriger Flüssigkeit von 37°C im wesentlichen unlöslich und zusammenhängend bleibt und nicht auf einfache Weise so dispergiert werden kann, dass eine parenterale, insbesondere intravenöse, gefahrlose Applikation möglich ist. Vorzugsweise beträgt die Dauer der visuellen Unterscheidbarkeit wenigstens eine Minute, vorzugsweise mindestens 10 Minuten.

Die Viskositätserhöhung des Extrakts führt dazu, dass dessen Nadelgängigkeit bzw. Spritzbarkeit erschwert oder sogar unmöglich gemacht wird. Sofern das Gel visuell unterscheidbar bleibt, bedeutet dies, dass das erhaltene Gel beim Einbringen in eine weitere Menge wässriger Flüssigkeit, z.B. durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, dass eine parenterale, insbesondere intravenöse, Applikation gefahrlos möglich ist. In Kombination mit mindestens einer ggf. vorhandenen Komponente (a) bis (e) führt dies zusätzlich zu unangenehmen Brennen, Erbrechen, schlechtem Geschmack und/oder zur visuellen Abschreckung.

Eine intravenöse Applikation eines entsprechenden Gels würde daher mit großer Wahrscheinlichkeit zur Verstopfung von Gefäßen, verbunden mit schweren gesundheitlichen Schäden des Missbrauchers führen.

Zur Überprüfung, ob ein viskositätserhöhendes Mittel als Komponente (b) zur Anwendung in der erfindungsgemäß erhaltenen Darreichungsform geeignet ist, wird der Wirkstoff mit dem viskositätserhöhenden Mittel gemischt und in 10 ml Wasser bei einer Temperatur von 25 °C suspendiert. Bildet sich hierbei ein Gel, welches den obenstehend genannten Bedingungen genügt, eignet sich das entsprechende viskositätserhöhende Mittel zur zusätzlichen Missbrauchs-Vorbeugung bzw. - Verhinderung bei den erfindungsgemäß erhaltenen Darreichungsformen.

Sofern der erfindungsgemäß erhaltenen Darreichungsform die Komponente (b) hinzugefügt wird, kommen vorzugsweise eine oder mehrere viskositätserhöhende Mittel zum Einsatz, die ausgewählt sind aus der Gruppe umfassend mikrokristalline Cellulose mit 11 Gew.-% Carboxymethylcellulose-Natrium (Avicel^{®} RC 591), Carboxymethylcellulose-Natrium (Blanose^{®}, CMC-Na C300P^{®}, Frimulsion BLC-5^{®}, Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Pektine, vorzugsweise aus Citrusfrüchten oder Äpfeln (Cesapectin^{®} HM Medium Rapid Set), Wachsmaisstärke (C*Gel 04201^{®}), Natriumalginat (Frimulsion ALG (E401)^{®}), Guarkernmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150^{®}), Tarakemmehl (Polygum 43/1^{®}), Propylenglykoalginat (Protanal-Ester SD-LB^{®}),, Natrium-Hyaluronat, Tragant, Taragummi (Vidogum SP 200^{®}), fermentiertes Polysaccharid- Welan Gum (K1A96), Xanthan-Gummi (Xantural 180^{®}). Xanthane sind besonders bevorzugt. Die in Klammern angegebenen Bezeichnungen sind die Handelsnamen, unter denen die jeweiligen Materialien am Markt geführt sind. Im allgemeinen ist eine Menge von 0,1 bis 20 Gew.%, besonders bevorzugt 0,1 bis 15 Gew.%, bezogen auf das Gesamtgewicht der Darreichungsform, der/des genannten viskositätserhöhenden Mittels ausreichend, um die vorstehend genannten Bedingungen zu erfüllen.

Die viskositätserhöhenden Mittel der Komponente (b), sofern vorgesehen, liegen in der erfindungsgemäß erhaltenen Darreichungsform bevorzugt in Mengen von ≥ 5 mg pro Darreichungseinheit, d.h. pro Dosiereinheit vor.

In einer besonders bevorzugten Ausführungsform der vorliegenden Erfindung kommen als Komponente (b) solche viskositätserhöhenden Mittel zum Einsatz, die bei der Extraktion aus der Darreichungsform mit der notwendigen Mindestmenge an wässriger Flüssigkeit ein Gel bilden, das Luftblasen einschließt. Die so erhaltenen Gele zeichnen sich durch ein trübes Erscheinungsbild aus, durch das der potentielle Missbraucher zusätzlich optisch gewarnt und von dessen parenteraler Applikation abgehalten wird.

Die Komponente (C) kann auch gegebenenfalls als zusätzliches viskositätserhöhendes Mittel dienen, das mit Hilfe einer notwendigen Mindestmenge einer wässrigen Flüssigkeit, ein Gel bildet.

Es ist auch möglich, die viskositätserhöhenden Mittel und die übrigen Bestandteile in räumlich voneinander getrennter Anordnung in der erfindungsgemäße erhaltenen Darreichungsform zu formulieren.

Des weiteren kann die erfindungsgemäß erhaltene Darreichungsform zur Vorbeugung und Sicherung gegen Missbrauch die Komponente (c) aufweisen, nämlich einen oder mehrere Antagonisten für den Wirkstoff bzw. die Wirkstoffe mit Missbrauchspotential, wobei die Antagonistenmenge vorzugsweise räumlich getrennt von den übrigen Bestandteilen der erfindungsgemäß erhaltenen Darreichungsform vorliegen und keine Wirkung bei bestimmungsgemäßer Verwendung entfalten.

Geeignete Antagonisten zur Verhinderung des Missbrauchs der Wirkstoffe sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Sofern der in der Darreichungsform vorliegende Wirkstoff ein Opioid ist, kommt als Antagonist bevorzugt ein Antagonist ausgewählt aus der Gruppe umfassend Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten, sofern eine Ausrüstung mit der Komponente (c) vorgesehen ist, in einer Menge von ≥ 1 mg, besonders bevorzugt in einer Menge von 3 bis 100 mg, ganz besonders bevorzugt in einer Menge von 5 bis 50 mg auf pro Darreichungsform, d.h. pro Dosiereinheit eingesetzt.

Weist die erfindungsgemäß erhaltene Darreichungsform als Wirkstoff ein Stimulanz auf, ist der Antagonist bevorzugt ein Neuroleptikum, vorzugsweise wenigstens eine Verbindung ausgewählt aus der Gruppe bestehend aus Haloperidol, Promethacin, Fluophenozin, Perphenazin, Levomepromazin, Thioridazin, Perazin, Chtorpromazin, Chlorprotheaxin, Zucklopantexol, Flupentexol, Prithipendyl, Zotepin, Penperidol, Piparmeron, Melperol und Bromperidol.

Vorzugsweise weist die erfindungsgemäß erhaltene Darreichungsform diese Antagonisten in einer üblichen, dem Fachmann bekannten therapeutischen Dosierung, besonders bevorzugt in einer gegenüber der üblichen Dosierung verdoppelten bis verdreifachten Menge pro Dosiereinheit auf.

Sofern die Kombination zur weiteren Vorbeugung und Sicherung der erfindungsgemäß hergestellten Darreichungsform gegen Missbrauch die Komponente (d) mitumfasst, kann sie wenigstens ein Emetikum aufweisen, das vorzugsweise in einer räumlich getrennten Anordnung von den übrigen Komponenten der erfindungsgemäß hergestellten Darreichungsform vorliegen und bei bestimmungsgemäßer Anwendung keine Wirkung im Körper entfalten sollte.

Geeignete Emetika zur zusätzlichen Verhinderung des Missbrauchs eines Wirkstoffs sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäß erhaltenen Darreichungsform vorliegen.

In der erfindungsgemäß erhaltenen Darreichungsform kann bevorzugt ein Emetikum auf Basis eines oder mehrerer inhaltsstoffe von Radix Ipecacuanhae (Brechwurzel), vorzugsweise auf Basis des Inhaltsstoffes Emetin, in Betracht, wie sie z.B. in pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden.

Vorzugsweise kann die erfindungsgemäß erhaltene Darreichungsform als Komponente (d) das Emetikum Emetin aufweisen, bevorzugt in einer Menge von ≥ 3 mg, besonders bevorzugt ≥ 10 mg und ganz besonders bevorzugt in einer Menge von ≥ 20 mg pro Darreichungsform, d. h. Dosiereinheit.

Ebenfalls bevorzugt kann als Emetikum Apomorphin als zusätzliche Missbrauchssicherung zum Einsatz kommen, vorzugsweise in einer Menge von vorzugsweise ≥ 3 mg, besonders bevorzugt ≥ 5 mg und ganz besonders bevorzugt ≥ 7 mg pro Dosiereinheit.

Sofern die erfindungsgemäß erhaltene Darreichungsform die Komponente (e) als weiteren missbrauchsverhindernden Hilfsstoff enthält, so wird durch den Einsatz eines solchen Farbstoffes, insbesondere bei dem Versuch, den Wirkstoff für eine parenterale, vorzugsweise intravenöse Applikation, zu extrahieren, eine intensive Farbgebung einer entsprechenden wässrigen Lösung hervorgerufen, die zur Abschreckung beim potentiellen Missbraucher führen kann. Auch ein oraler Missbrauch, der üblicherweise über eine wässrige Extraktion des Wirkstoffes eingeleitet wird, kann durch diese Farbgebung verhindert werden. Geeignete Farbstoffe sowie die für die notwendige Abschreckungswirkung erforderlichen Mengen sind der WO 03/015531 zu entnehmen.

Sofern die erfindungsgemäß erhaltene Darreichungsform als zusätzlichen Missbrauchs-verhindernden Hilfsstoff die Komponente (f) enthält, so wird durch diesen Zusatz von wenigstens einem Bitterstoff durch die damit eintretende Geschmacksverschlechterung der Darreichungsform der orale und/oder nasale Missbrauch zusätzlich verhindert.

Geeignete Bitterstoffe sowie die für den Einsatz wirksamen Mengen sind der US-2003/0064099 A1 zu entnehmen. Vorzugsweise eignen sich als Bitterstoffe Aromaöle, vorzugsweise Pfefferminzöl, Eukalyptusöl, Bittermandelöl, Menthol, Fruchtaromastoffe, vorzugsweise Aromastoffe von Zitronen, Orangen, Limonen, Grapefruit oder Mischungen davon, und/oder Denatonium-Benzoat (Bitrox®). Besonders bevorzugt ist Denatonium-Benzoat.

Die erfindungsgemäß erhaltene, feste Darreichungsform eignet sich nicht nur zur oralen, sondern auch zur vaginalen oder rektalen Anwendung, vorzugsweise aber zur oralen Einnahme. Vorzugsweise ist sie nicht filmförmig. Die erfindungsgemäße Darreichungsform kann in multipartikulärer Form, bevorzugt in zylindrischer Form, in Form von Mikrotabletten, Mikrokapseln, Mikropellets, Granulaten, Sphäroiden, Perlen oder Pellets, ggf. in Kapseln abgefüllt oder zu Tabletten verpresst, vorzugsweise zur oralen Verabreichung, vorliegen. Vorzugsweise weisen die multipartikulären Formen eine Größe bzw. Größenverteilung im Bereich von 0,1 bis 3 mm, besonders bevorzugt im Bereich von 0,5 bis 2 mm auf. Je nach gewünschter Darreichungsform *wird wenigstens ein Antioxidans als Hilfsstoff (B) und ggf. weitere übliche Hilfsstoffe (B) zur Formulierung der Darreichungsform mitverwendet.*

In einer weiteren bevorzugten Ausführungsform liegt die erfindungsgemäß erhaltene Darreichungsform in Form einer Tablette, einer Kapsel oder in Form eines oralen osmotischen therapeutischen Systems (OROS) vor, vorzugsweise wenn mindestens noch eine weitere missbrauchsverhindernde Komponente (a) - (f) vorhanden ist.

Sofern die Komponenten (c) und/oder (d) und/oder (f) in der erfindungsgemäß erhaltenen Darreichungsform vorhanden sind, ist darauf zu achten, dass sie so formuliert oder so gering dosiert sind, dass sie bei bestimmungsgemäßer Applikation der Darreichungsform praktisch keine den Patienten oder die Wirksamkeit des Wirkstoffs beeinträchtigende Wirkung entfalten können.

Sofern die erfindungsgemäß erhaltene Darreichungsform die Komponente (d) und/oder (f) enthält, ist die Dosierung so zu wählen, dass bei bestimmungsgemäßer oraler Applikation keine negative Wirkung hervorgerufen wird. Wird jedoch die vorgesehene Dosierung bei einem Missbrauch überschritten, wird Übelkeit bzw. Brechreiz bzw. schlechter Geschmack hervorgerufen. Die jeweilige Menge der Komponente (d) und/oder (f), die vom Patienten bei bestimmungsgemäßer oraler Applikation noch toleriert wird, kann vom Fachmann durch einfache Vorversuche ermittelt werden.

Sofern aber unabhängig von der praktisch nicht möglichen Pulverisierbarkeit der erfindungsgemäß hergestellten, weiteren Darreichungsform zur Sicherung der Darreichungsform der Einsatz der Komponenten (c) und/oder (d) und/oder (f) vorgesehen ist, sollten diese Komponenten bevorzugt in einer so hohen Dosierung zum Einsatz kommen, dass sie bei einer missbräuchlichen Applikation der Darreichungsform eine intensive negative Wirkung beim Missbraucher hervorrufen. Dies gelingt vorzugsweise durch eine räumliche Trennung zumindest des Wirkstoffes bzw. der Wirkstoffe von den Komponenten (c) und/oder (d) und/oder (f), wobei bevorzugt der Wirkstoff bzw. die Wirkstoffe in wenigstens einer Untereinheit (X) und die Komponenten (c) und/oder (d) und/oder (f) in wenigstens einer Untereinheit (Y) vorliegen, und wobei die Komponenten (c), (d) und (f) bei bestimmungsgemäßer Applikation der Darreichungsform bei Einnahme und/oder im Körper nicht ihre Wirkung entfalten und die übrigen Formulierungskomponenten insbesondere die Komponente (C) und ggf. (D) identisch sind.

Sofern die erfindungsgemäß erhaltene Darreichungsform wenigstens 2 der Komponenten (c) und (d) bzw. (f) aufweist, können diese jeweils in derselben oder in verschiedenen Untereinheiten (Y) vorliegen. Vorzugsweise liegen, sofern vorhanden, alle Komponenten (c) und (d) und (f) in ein- und derselben Untereinheit (Y) vor.

Untereinheiten im Sinne der vorliegenden Erfindung sind feste Formulierungen, die jeweils neben üblichen, dem Fachmann bekannten Hilfsstoffen den (die) Wirkstoff(e), mindestens ein Polymer (C) und die gegebenenfalls vorhandene Komponente (D) und gegebenenfalls wenigstens eine der gegebenenfalls vorhandenen Komponenten (a) und/oder (b) und/oder (e) bzw. jeweils wenigstens ein Polymer (C) und gegebenenfalls (D) und den (die) Antagonist(en) und/oder das Emetikum (die Emetika) und/oder die Komponente (e) und/oder die Komponente (f) und gegebenenfalls wenigstens eine der gegebenenfalls vorhandenen Komponenten (a) und/oder (b) enthalten. Dabei ist darauf zu achten, dass jede der genannten Untereinheiten nach den vorstehend angegebenen, erfindungsgemäßen Verfahren formuliert werden.

Ein wesentlicher Vorteil der getrennten Formulierung der Wirkstoffe von den Komponenten (c) bzw. (d) bzw. (f) in Untereinheiten (X) und (Y) der erfindungsgemäß hergestellten Darreichungsform besteht darin, dass bei ihrer bestimmungsgemäßen Applikation die Komponenten (c) und/oder (d) und/oder (f) bei Einnahme und/oder im Körper praktisch nicht freigesetzt werden oder nur in so geringen Mengen freigesetzt werden, dass sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist. Vorzugsweise werden die Komponenten (c) und/oder (d) und/oder (f) bei bestimmungsgemäßer Applikation der Darreichungsform im Körper des Patienten praktisch nicht freigesetzt oder vom Patienten nicht wahrgenommen.

Der Fachmann versteht, dass diese vorstehend genannten Bedingungen in Abhängigkeit von den jeweils eingesetzten Komponenten (c), (d) und/oder (f) sowie der Formulierung der Untereinheiten bzw. der Darreichungsform variieren können. Die für die jeweilige Darreichungsform optimale Formulierung kann durch einfache Vorversuche ermittelt werden. Entscheidend ist, dass die jeweiligen Untereinheiten das Polymer (C) und gegebenenfalls die Komponente (D) enthalten und in der vorstehend angegebenen Weise formuliert und erfindungsgemäß hergestellt wurden.

Sollte es den Missbrauchem wider Erwarten gelingen, eine solche erfindungsgemäß hergestellte Darreichungsform, welche die Komponenten (c) und/oder (e) und/oder (d) und/oder (f) in Untereinheiten (Y) aufweist, zum Zwecke der missbräuchlichen Einnahme des Wirkstoffes zu zerkleinern und ein Pulver zu erhalten, das mit einem geeigneten Extraktionsmittel extrahiert wird, wird neben dem Wirkstoff auch die jeweilige Komponente (c) und/oder (e) und/oder (f) und/oder (d) in einer Form erhalten, in der sie von dem Wirkstoff nicht auf einfache Weise zu separieren ist, so dass sie bei der Applikation der manipulierten Darreichungsform, insbesondere bei oraler und/oder parenteraler Verabreichung, ihre Wirkung bei Einnahme und/oder im Körper entfaltet und zusätzlich eine der Komponente (c) und/oder (d) und/oder (f) entsprechende negative Wirkung beim Missbraucher hervorruft oder ein Versuch, den Wirkstoff zu extrahieren durch die Farbgebung abschreckt und so den Missbrauch der Darreichungsform verhindert.

Die erfindungsgemäße Formulierung einer Darreichungsform, in der eine räumliche Trennung des Wirkstoffes bzw. der Wirkstoffe von den Komponenten (c), (d) und/oder (e), vorzugsweise durch Formulierung in verschiedenen Untereinheiten erfolgt ist, kann in vielfältiger Art und Weise erfolgen, wobei die entsprechenden Untereinheiten in der Darreichungsform jeweils in beliebiger räumlicher Anordnung zueinander vorliegen können, sofern die vorstehend genannten Bedingungen für die Freisetzung der Komponenten (c) und/oder (d) erfüllt sind.

Der Fachmann versteht, dass die ggf. auch vorliegenden Komponente(n) (a) und/oder (b) bevorzugt sowohl in den jeweiligen Untereinheiten (X) und (Y) als auch in Form von eigenständigen, den Untereinheiten (X) und (Y) entsprechenden Untereinheiten in der erfindungsgemäß hergestellten Darreichungsform formuliert werden können, so lange die Sicherung der Darreichungsform gegen den Missbrauch wie auch die Wirkstofffreisetzung bei bestimmungsgemäßer Applikation durch die Art der Formulierung nicht beeinträchtig werden und das Polymer (C) und gegebenenfalls (D) mit formuliert und die Formulierung gemäß den vorstehend angegebenen Verfahren zur Erzielung der notwendigen Härte durchgeführt wird.

In einer bevorzugten Ausführungsform der erfindungsgemäß hergestellten Darreichungsform liegen die Untereinheiten (X) und (Y) in multipartikulärer Form vor, wobei Mikrotabletten, Mikrokapseln, Mikropellets, Granulaten, Sphäroiden, Perlen oder Pellets bevorzugt sind und sowohl für die Untereinheit (X) als auch (Y) dieselbe Form, d.h. Gestaltung gewählt wird, damit keine Separierung der Untereinheiten (X) von (Y), z. B. durch mechanische Auslese, möglich ist. Die multipartikulären Formen weisen bevorzugt eine Größe im Bereich von 0,1 bis 3 mm, vorzugsweise 0,5 bis 2 mm auf.

Die Untereinheiten (X) und (Y) in multipartikulärer Form können auch bevorzugt in eine Kapsel abgefüllt oder zu einer Tablette verpresst werden, wobei die jeweiligen Endformulierungen dergestalt erfolgen, dass die Untereinheiten (X) und (Y) auch in der resultierenden Darreichungsform erhalten bleiben.

Die jeweiligen multipartikulären Untereinheiten (X) bzw. (Y) mit identischer Formgebung sollten auch nicht visuell voneinander unterscheidbar sein, damit sie vom Missbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z. B. die Retardierung eines oder mehrerer Wirkstoffe oder eine magensaftresistente Ausrüstung der jeweiligen Untereinheiten.

Die multipartikulären Untereinheiten können auch als Slurry oder als Suspension in pharmazeutisch unbedenklichen Suspensionsmedien als orale Darreichungsform formuliert werden.

In einer weiteren bevorzugten Ausführungsform der vorliegenden Erfindung sind die Untereinheiten (X) und (Y) jeweils schichtförmig zueinander angeordnet.

Bevorzugt sind hierfür die schichtförmigen Untereinheiten (X) und (Y) in der erfindungsgemäß hergestellten Darreichungsform vertikal oder horizontal zueinander angeordnet, wobei jeweils auch eine oder mehrere schichtförmige Untereinheiten (X) und eine oder mehrere schichtförmige Untereinheiten (Y) in der Darreichungsform vorliegen können, so daß neben den bevorzugten Schichtenfolgen (X)-(Y) bzw. (X)-(Y)-(X) beliebige andere Schichtenfolgen in Betracht kommen, ggf. in Kombination mit Schichten enthaltend die Komponenten (a) und/oder (b).

Ebenfalls bevorzugt ist eine erfindungsgemäß hergestellte Darreichungsform, in der die Untereinheit (Y) einen Kern bildet, der von der Untereinheit (X) vollständig umhüllt wird, wobei zwischen diesen Schichten eine Trennschicht (Z) vorhanden sein kann. Ein entsprechender Aufbau eignet sich bevorzugt auch für die vorstehend genannten multipartikulären Formen, wobei dann beide Untereinheiten (X) und (Y) sowie eine ggf. vorhandene Trennschicht (Z), die der erfindungsgemäßen Härteanforderung genügen muss, in ein- und derselben multipartikulären Form formuliert sind.

In einer weiteren bevorzugten Ausführungsform der erfindungsgemäß hergestellten Darreichungsform bildet die Untereinheit (X) einen Kern, der von der Untereinheit (Y) umhüllt wird, wobei letztere wenigstens einen Kanal aufweist, der von dem Kern an die Oberfläche der Darreichungsform führt.

Zwischen einer Schicht der Untereinheit (X) und einer Schicht der Untereinheit (Y) kann die erfindungsgemäß hergestellte Darreichungsform jeweils eine oder mehrere, vorzugsweise eine, ggf. quellbare Trennschicht (Z) zur räumlichen Trennung der Untereinheit (X) von (Y) aufweisen.

Sofern die erfindungsgemäß hergestellte Darreichungsform die schichtförmigen Untereinheiten (X) und (Y) sowie eine ggf. vorhandene Trennschicht (Z) in einer zumindest teilweise vertikalen oder horizontalen Anordnung aufweist, liegt sie bevorzugt in Form einer Tablette oder eines Laminats vor.

Hierbei kann in einer besonders bevorzugten Ausführungsform die freie Oberfläche der Untereinheit (Y) vollständig und ggf. zumindest ein Teil der freien Oberfläche der Untereinheit(en) (X) und ggf. zumindest ein Teil der freien Oberfläche der ggf. vorhandenen Trennschicht(en) (Z) mit wenigstens einer die Freisetzung der Komponente (c) und/oder (e) und/oder (d) und/oder (f) verhindernden Barriereschicht (Z') überzogen sein. Auch die Barriereschicht (Z') muss die erfindungsgemäßen Härtevoraussetzungen erfüllen.

Ebenfalls besonders bevorzugt ist eine Ausführungsform der erfindungsgemäß hergestellten Darreichungsform, die eine vertikale oder horizontale Anordnung der Schichten der Untereinheiten (X) und (Y) und wenigstens eine dazwischen angeordnete Push-Schicht (p) sowie ggf. eine Trennschicht (Z) aufweist, in der sämtliche freie Oberflächen des aus den Untereinheiten (X) und (Y), der Push-Schicht und der ggf. vorhandenen Trennschicht (Z) bestehenden Schichtaufbaus mit einem semipermeablen Überzug (E) ausgerüstet sind, der für ein Freisetzungsmedium, d. h. üblicherweise eine physiologische Flüssigkeit, durchlässig, für den Wirkstoff und für die Komponente (c) und/oder (d) und/oder (f) im wesentlichen undurchlässig ist, und wobei dieser Überzug (E) im Bereich der Untereinheit (X) wenigstens eine Öffnung zur Freisetzung des Wirkstoffes aufweist.

Eine entsprechende Darreichungsform ist dem Fachmann beispielsweise unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt.

In einer weiteren bevorzugten Ausführungsform hat die Untereinheit (X) der erfindungsgemäß hergestellten Darreichungsform die Form einer Tablette, deren Steg und ggf. eine der beiden Grundflächen mit einer die Komponente (c) und/oder (d) und/oder (f) enthaltenden Barriereschicht (Z') bedeckt ist.

Der Fachmann versteht, dass die bei der erfindungsgemäßen Formulierung der Darreichungsform jeweils zum Einsatz kommenden Hilfsstoffe der Untereinheit(en) (X) bzw. (Y) sowie ggf. der vorhandenen Trennschicht(en) (Z) und/oder der Barriereschicht(en) (Z') in Abhängigkeit von deren Anordnung in der Darreichungsform, der Applikationsart sowie in Abhängigkeit von dem jeweiligen Wirkstoff der ggf. vorhandenen Komponenten (a) und/oder (b) und/oder (e) und der Komponente (c) und/oder (d) und/oder (f) variieren. Die Materialien, die über die jeweils erforderlichen Eigenschaften verfügen sind, dem Fachmann an sich bekannt.

Sofern die Freisetzung der Komponente (c) und/oder (d) und/oder (f) aus der Untereinheit (Y) der erfindungsgemäß hergestellten Darreichungsform mit Hilfe einer Umhüllung, vorzugsweise einer Barriereschicht, verhindert wird, kann die Untereinheit aus üblichen, dem Fachmann bekannten Materialien bestehen, sofern sie wenigstens ein Polymer (C) und gegebenenfalls (D) zur Erfüllung der Härtebedingung enthält und erfindungsgemäß hergestellt wurde.

Ist eine entsprechende Barriereschicht (Z') zur Verhinderung der Freisetzung der Komponente (c) und/oder (d) und/oder (f) nicht vorgesehen, sind die Materialien der Untereinheiten so zu wählen, dass eine Freisetzung der jeweiligen Komponente (c) und/oder (d) aus der Untereinheit (Y) praktisch ausgeschlossen ist.

Bevorzugt können hierzu die nachstehend aufgeführten Materialien zum Einsatz kommen, die auch für den Aufbau der Barriereschicht geeignet sind.

Bevorzugte Materialien sind solche, die ausgewählt sind aus der Gruppe umfassend Alkylcellulosen, Hydroxyalkylcellulosen, Glucanen, Skleroglucanen, Mannanen, Xanthanen, Copolymeren aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane sowie Polyurethane und deren Copolymeren.

Besonders geeignete Materialien können ausgewählt werden aus der Gruppe umfassend Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe umfassend Copolymere aus Butylmethacrylat und Isobutylmethacrylat, Copolymere aus Methylvinylether und Maleinsäure mit erhöhtem Molekulargewicht, Copolymere aus Methylvinylether und Maleinsäuremonoethylester, Copolymere aus Methylvinylether und Maleinsäureanhydrid sowie Copolymere aus Vinylalkohol und Vinylacetat.

Weitere, zur Formulierung der Barriereschicht besonders geeignete Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1).

Ggf. können die vorstehend genannten Materialien mit weiteren üblichen, dem Fachmann bekannten Hilfsstoffen, *erfindungsgemäß wenigstens ein Antioxidans als Hilfsstoff (B) und gegebenenfalls weitere Hilfsstoffe (B) ausgewählt aus der Gruppe umfassend Weichmacher und Gleitmittel* wie z. B. Glycerinmonostearat, halbsynthetische Triglyceridderivate, halbsynthetische Glyceride, hydriertes Rizinusöl, Glycerinpalmitostearat, Glycerinbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierte Triglyceride, Glyceride, Polyoxyalkylenglykole, Polyalkylenglykole und deren Derivate abgemischt werden.

Sofern die erfindungsgemäß hergestellte Darreichungsform eine Trennschicht (Z') aufweist, kann diese, ebenso wie die nicht umhüllte Untereinheit (Y) vorzugsweise aus den vorstehend, für die Barriereschicht beschriebenen Materialien bestehen. Der Fachmann versteht, daß auch über die Dicke der Trennschicht die Freisetzung des Wirkstoffes bzw. der Komponente (c) und/oder (d) aus der jeweiligen Untereinheit gesteuert werden kann.

Die erfindungsgemäß hergestellte Darreichungsform weist eine kontrollierte Freisetzung des Wirkstoffes auf. Sie eignet sich dabei vorzugsweise für eine 2x tägliche Verabreichung an Patienten.

Die erfindungsgemäß hergestellte Darreichungsform kann einen oder mehrere Wirkstoffe mit Missbrauchspotential zumindest teilweise in einer darüber hinaus retardierten Form aufweisen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge. Die Wirkstoffabgabe muss aber so gesteuert sein, daß die vorstehend genannten Bedingungen jeweils erfüllt sind, z.B. das bei bestimmungsgemäßer Applikation der Darreichungsform der Wirkstoff bzw. die Wirkstoffe praktisch komplett freigesetzt wird, bevor die ggf. vorhandenen Komponente (c) und/oder (d) eine beeinträchtigende Wirkung entfalten können. Außerdem darf durch die Zugabe von retardierenden Materialien keine Beeinträchtigung der notwendigen Härte erfolgen.

Die kontrollierte Freisetzung aus der erfindungsgemäß erhaltenen Darreichungsform wird vorzugsweise durch Einbettung des Wirkstoffes in eine Matrix erzielt. Die als Matrixmaterialien dienenden Hilfsstoffe kontrollieren die Wirkstofffreisetzung. Matrixmaterialien können beispielweise hydrophile, gelbildende Materialien sein, woraus die Wirkstofffreisetzung hauptsächlich durch Diffusion erfolgt, oder hydrophobe Materialien sein, woraus die Wirkstofffreisetzung hauptsächlich durch Diffusion aus den Poren in der Matrix erfolgt.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophoben Polymeren, Wachsen, Fetten, langkettigen Fettsäuren, Fettalkoholen oder entsprechenden Estern oder Ethern oder deren Gemische. Besonders bevorzugt werden als hydrophobe Materialien Mono- oder Digliceride von C12-C30-Fettsäuren und/oder C12-C30-Fettalkohole und/oder Wachse oder deren Gemische eingesetzt.

Es ist auch möglich, Mischungen der vorstehend genannten hydrophilen und hydrophoben Materialien als Matrixmaterialien einzusetzen.

Des weiteren können auch die Komponenten (C) und ggf. vorhandene Komponente (D), die zur Erzielung der erfindungsgemäß notwendigen Bruchfestigkeit von mindestens 500 N dienen, bereits als zusätzliche Matrixmaterialien dienen.

Sofern die erfindungsgemäß hergestellte Darreichungsform zur oralen Applikation vorgesehen ist, kann sie bevorzugt auch einen magensaftresistenten Überzug aufweisen, der sich in Abhängigkeit vom pH-Wert der Freisetzungsumgebung auflöst. Durch diesen Überzug kann erreicht werden, dass die erfindungsgemäß hergestellte Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf.

Entsprechende Materialien und Verfahren zur Retardierung von Wirkstoffen sowie zum Aufbringen magensaftresistenter Überzüge sind dem Fachmann beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt.

### Methode zur Bestimmung der Bruchfestigkeit

Zur Überprüfung, ob ein Material als Komponente (C) oder (D) eingesetzt werden kann, wird das Material in einer Tablettenform mit Hilfe eines Lösungsmittels für die Komponente (C) oder (D) gelöst und nach Entfernung des Lösungsmittels bei Temperaturen unterhalb des Erweichungspunktes des Materials zu einer Tablette mit einem Durchmesser von 10 mm und einer Höhe von 5 mm mit einer Kraft von 150 N verpresst.

Mit so hergestellten Tabletten wird gemäß der Methode zur Bestimmung der Bruchfestigkeit von Tabletten, veröffentlicht im Europäischen Arzneibuch 1997, Seite 143,144, Methode Nr. 2.9.8. unter Einsatz der nachstehend aufgeführten Apparatur die Bruchfestigkeit bestimmt. Als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

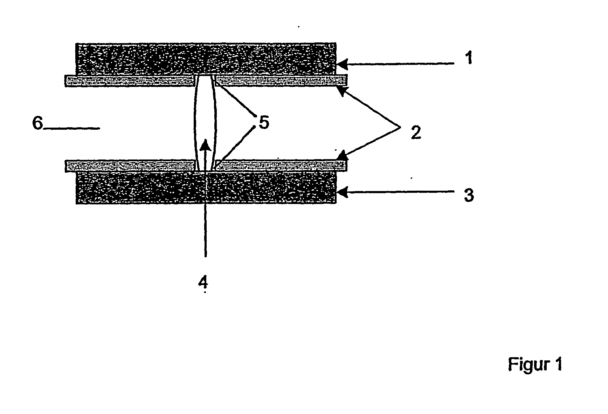

**Figur 1** zeigt die Messung der Bruchfestigkeit einer Tablette, insbesondere die dafür eingesetzte Justierungsvorrichtung (6) der Tablette (4) vor und während der Messung. Darzu wird die Tablette (4) zwischen der oberen Druckplatte (1) und der unteren Druckplatte (3) der nicht dargestellten Vorrichtung zur Kraftaufbringung mit Hilfe von zwei 2-teiligen Einspannvorrichtungen, die jeweils mit der oberen bzw. unteren Druckplatte nach Einstellung des zur Aufnahme und zur Zentrierung der zu messenden Tablette notwendigen Abstands (5) fest verbunden (nicht dargestellt) werden. Zur Einstellung des Abstands (5) können die 2-teiligen Einspannvorrichtungen jeweils auf der Druckplatte, auf der sie gelagert sind, horizontal nach außen oder innen bewegt werden.

Als bruchfest bei einer bestimmten Krafteinwirkung werden auch die Tabletten eingestuft, bei denen kein Bruch feststellbar, aber ggf. eine plastische Verformung der Tablette durch die Krafteinwirkung erfolgt ist.

Bei den erfindungsgemäß erhaltenen Darreichungsformen wird die Bruchfestigkeit nach der aufgeführten Meßmethode zur Bestimmung der Bruchfestigkeit bestimmt, wobei von Tabletten abweichenden Darreichungsformen ebenso geprüft werden.

Im Folgenden wird die Erfindung anhand von Beispielen erläutert. Diese Erläuterungen sind lediglich beispielhaft und schränken den allgemeinen Erfindungsgedanken nicht ein.

### Beispiele

### Beispiel 1

| | Pro Tablette | Gesamtansatz |
|---|---|---|
| Tramadol HCl | 100,0 mg | 1495,0 g |
| Polyethylenoxid, MG 7 000 000 (Polyox WSR 303 Fa. Dow) | 167,8 mg | 2508,6 g |
| Hydroxypropylmethylcellulose (Hypromellose 100 000 mPa) | 33,5 mg | 500,8 g |
| Butylhydroxytoluol (BHT) | 0,2 mg | 3,0 g |
| Gesamtmasse | 300,5 mg | 4507,4 g |

Die angegebene BHT-Menge wurde in Ethanol (96%) gelöst, so dass man eine 7,7%-ige (m/m) ethanolische Lösung erhält. Diese wurde zunächst mit dem 150 g Polyethylenoxid in einem Schnellmischer für 30 Minuten gemischt und dann die restliche Menge Polyethylenoxid hinzugegeben und erneut für 30 Minuten gerührt. Die Masse wird bei 40°C für 12 h getrocknet.

Alle weiteren Komponenten wurden zugesetzt und in einem Freifallmischer für 15 Minuten gemischt. Die Pulvermischung wurde auf Formen, die jeweils einen Durchmesser von 13 mm und einer Tiefe von 6 mm verteilt. Mit Hilfe einer Kanülenspritze wurde die Mischung jeweils in 0,5 ml Ethanol 96% suspendiert und anschließend jeweils mit 0,5 ml dest. Wasser versetzt. Nach 24 Stunden Quellzeit wurde die gequollene Masse 24 h bei 40°C getrocknet.

Die aufgeteilten, getrockneten Massen wurden jeweils mit Hilfe einer Ekzenterpresse vom Typ EK 0 zu Tabletten verpresst. Das Tablettierwerkzeug hatte einen Durchmesser von 10 mm und einem Wölbungsradius von 8 mm.

Die Bruchfestigkeit der Tabletten wurde nach der vorstehend beschriebenen Methode bestimmt. Bei der Krafteinwirkung von 500 N trat kein Bruch auf. Die Tabletten konnten weder mit einem Hammer noch mit Hilfe von Mörser und Pistill zerkleinert werden.

Die in vitro Freisetzung des Wirkstoffs aus den Tabletten wurde in der Blattrührerapparatur mit Sinker nach Pharm. Eur. bestimmt. Die Temperatur des Freisetzungsmediums betrug 37°C und die Umdrehungsgeschwindigkeit des Rührers 75 min⁻¹. Als Freisetzungsmedium wurde 600 ml Darmsaft pH 6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min. | 20% |
| 240 min | 43% |
| 480 min | 83% |
| 720 min | 90% |

### Beispiel 2

| **Pulvermischung** | Gesamtansatz | Pro Tablette |
|---|---|---|
| Tramadol HCl | 100,1 g | 100 mg |
| Polyethylenoxid | 300,0 g | 299,7 mg |
| MG 5000 000 (Polyox WSR Coagulant, Fa. Dow), | | |
| Hydroxypropylmethylcellulose (Hypromellose 100 000 mPa) | 50,05 g | 50,0 mg |
| Butylhydroxytoluol (BHT) | 0,25 g | 0,25 mg |
| **Schaum** | | |
| Hydroxypropylmethylcellulose (Hypromellose 100 000 mPa) | 0,250 g | 0,25 mg |
| dest. Wasser | 49,8 g | |

Wie in Beispiel 1 angegeben, wurde zunächst die Pulvermischung hergestellt.

Zur Herstellung des Schaums wurde die angegebene Menge Hypromellose in dest. Wasser gelöst. Anschließend wurde mit einem Hochleistungsdispergiergerät, (IKA Ultraturax 25 Basic) ein Schaum hergestellt, indem zunächst 2 Minuten mit Stufe 1, dann 2 Minuten mit Mischergranulator bei Stufe 2 und schließlich 3 Minuten bei Stufe 3 gerührt wurde. In einem Mischer (Kenwood major classic 25 Basic) wurde der Pulvermischung unter ständigem Rühren der Schaum langsam zugegeben.

Anschließend wurde die granulierte Mischung 24 Stunden lang bei 40°C getrocknet und nach Siebung mit einem Sieb (Fa. Frewitt Typ GLA-A-ORV) mit 1 mm Öffnungen zu Tabletten mit einem Gewicht von 450,2 mg verpresst. Hierzu wurde eine Ekzenterpresse vom Typ EK 0 mit einem runden Tablettierwerkzeug mit einem Durchmesser von 10 mm und einem Wölbungsradius von 8 mm verwendet. Diese Tabletten wurden 1 Stunde lang bei 70°C getrocknet.

Die Bruchfestigkeit der Tabletten wurde nach der vorstehend angegebenen Methode bestimmt. Bei der Krafteinwirkung von 500 N trat kein Bruch auf. Die Tablette konnte weder mit einem Hammer noch mit Hilfe von Mörser und Pistill zerkleinert werden.

Die in vitro Freisetzung des Wirkstoffs aus den Tabletten wurde in der Blattrührerapparatur mit Sinker nach Pharm. Eur. bestimmt. Die Temperatur des Freisetzungsmediums betrug 37°C und die Umdrehungsgeschwindigkeit des Rührers 75 min⁻¹. Als Freisetzungsmedium wurde 600 ml Darmsaft pH 6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min. | 12% |
| 240 min | 47% |
| 480 min | 71% |
| 720 min | 84% |

## Patentansprüche

1. Verfahren zur Herstellung einer festen Darreichungsform mit zumindest vermindertem Missbrauchspotential, **dadurch gekennzeichnet, dass** man
a) zu einer Formulierungsmischung, enthaltend wenigstens einen Wirkstoff mit Missbrauchspotential (A), wenigstens ein Antioxidans als Hilfsstoff (B) und wenigstens ein synthetisches oder natürliches Polymer (C), das eine Bruchfestigkeit von mindestens 500 N aufweist, ein Lösungsmittel für das Polymer (C) zumindest in solchen Mengen zugibt, dass die Formulierungsmischung gleichmäßig befeuchtet wird;
b) gegebenenfalls die so zumindest befeuchtete Masse auf Teilmassen verteilt;
c) die Masse(n) trocknet; und
d) zu der Darreichungsform formt;
wobei Polymer (C) in solchen Mengen eingesetzt wird, dass auch die Darreichungsform eine Mindestbruchfestigkeit von mindestens 500 N aufweist.

2. Verfahren nach Anspruch 1, **dadurch gekennzeichnet, dass** die getrockneten Teilmassen jeweils der Masse einer Einheit der Darreichungsform entsprechen.

3. Verfahren nach Anspruch 1 oder 2, **dadurch gekennzeichnet, dass** die Formulierungsmischung vor der Zugabe des Lösungsmittels bereits in einem flüssigen Dispergierungsmittel, in dem die Polymerkomponente (C) nicht löslich ist, dispergiert wird.

4. Verfahren nach Anspruch 3, **dadurch gekennzeichnet, dass** die Formulierungsmasse vor oder nach ihrer Dispergierung bereits in Teilmassen jeweils entsprechend der Masse einer Einheit der Darreichungsform aufgeteilt wird.

5. Verfahren nach Anspruch 3 oder 4, **dadurch gekennzeichnet, dass** das Lösungsmittel und das Dispergierungsmittel miteinander mischbar sind.

6. Verfahren nach Anspruch 1, **dadurch gekennzeichnet, dass** das Lösungsmittel als Schaum in die Formulierungsmischung eingearbeitet wird.

7. Verfahren nach Anspruch 6, **dadurch gekennzeichnet, dass** der Schaum mit Hilfe von Schaumstabilisatoren stabilisiert ist.

8. Verfahren nach Anspruch 6 oder 7, **dadurch gekennzeichnet, dass** die mit dem Lösungsmittelschaum granulierte Masse getrocknet wird.

9. Verfahren nach Anspruch 8, **dadurch gekennzeichnet, dass** die getrocknete, granulierte Masse auf Teilmassen verteilt wird, die jeweils der Masse einer Einheit der Darreichungsform entsprechen und zu der Darreichungsform formt.

10. Verfahren nach Anspruch 1, **dadurch gekennzeichnet, dass** zu der Formulierungsmischung soviel Lösungsmittel zugegeben wird, dass eine formbare Paste erhalten wird.

11. Verfahren nach Anspruch 10, **dadurch gekennzeichnet, dass** die Paste vor oder nach ihrer Trocknung in Teilmassen aufgeteilt wird und die getrockneten Massen gegebenenfalls nach einer weiteren Verteilung jeweils auf eine Masse entsprechend der Masse einer Einheit der Darreichungsform zur Darreichungsform geformt oder umgeformt werden.

12. Verfahren nach Anspruch 11, **dadurch gekennzeichnet, dass** die Teilmassen die Form von Strängen aufweisen.

13. Verfahren nach Anspruch 12, **dadurch gekennzeichnet, dass** die Stränge mit Hilfe eines Siebes oder eines Strangformers erzeugt werden.

14. Verfahren nach Anspruch 12, **dadurch gekennzeichnet, dass** die getrockneten Stränge vereinzelt und zur Darreichungsform geformt werden.

15. Verfahren nach Anspruch 14, **dadurch gekennzeichnet, dass** die Formung mit Hilfe einer Tablettenpresse erfolgt.

16. Verfahren nach Anspruch 12, **dadurch gekennzeichnet, dass** die getrockneten Stränge mit Hilfe von Formwalzen oder mit Walzen ausgerüsteten Formbändern zur Darreichungsform geformt werden.

17. Verfahren nach Anspruch 11, **dadurch gekennzeichnet, dass** die Paste zu einem flächenförmigen Gebilde verarbeitet und getrocknet wird, aus dem die Darreichungsform ausgestanzt wird.

18. Verfahren nach den Ansprüchen 10 bis 12, **dadurch gekennzeichnet, dass** das Verfahren mit Hilfe eines Extruders durchgeführt wird.

19. Verfahren nach den Anspruch 1, **dadurch gekennzeichnet, dass** zu der Formulierungsmischung soviel Lösungsmittel zugegeben wird, dass zumindest die Polymerkomponente (C) gelöst wird.

20. Verfahren nach Anspruch 19, **dadurch gekennzeichnet, dass** die Lösung zu einem flächenförmigen Gebilde verarbeitet wird.

21. Verfahren nach Anspruch 20, **dadurch gekennzeichnet, dass** das flächenförmige Gebilde mit Hilfe eines Extruders mit Flachdüse oder durch Ausgießen der Lösung auf eine flächenförmige, ebene Unterlage erhalten wird.

22. Verfahren nach Anspruch 19 bis 21, **dadurch gekennzeichnet, dass** aus dem getrockneten, flächenförmigen Gebilde die Darreichungsform durch Stanzen geformt oder Kalandrieren erhalten wird.

23. Verfahren nach Anspruch 19, **dadurch gekennzeichnet, dass** die Lösung in solchen Teilmengen aufgeteilt wird, dass sie nach dem Trocknen jeweils der Masse einer Einheit der Darreichungsform entsprechen.

24. Verfahren nach Anspruch 23, **dadurch gekennzeichnet, dass** die Teilmengen in Formen entsprechend der Form einer Einheit der Darreichungsform abgefüllt werden.

25. Verfahren nach Anspruch 19, **dadurch gekennzeichnet, dass** die Lösung in beliebige Teilmengen aufgeteilt wird, die nach dem Trocknen gegebenenfalls wieder vereinigt zu der Darreichungsform geformt werden.

## Claims

1. Process for the production of a solid dosage form with at least reduced potential for abuse, **characterised in that**
a) to a formulation mixture containing at least one active ingredient with potential for abuse (A), at least one antioxidant as auxiliary substance (B) and at least one synthetic or natural polymer (C), which exhibits a breaking strength of at least 500 N, a solvent for the polymer (C) is added at least in such quantities that the formulation mixture is uniformly moistened;
b) the composition, which has been at least moistened thus is optionally divided into sub-portions;
c) the portion(s) are dried and
d) shaped into the dosage form,
wherein polymer (C) is used in such quantities that the dosage form also has a minimum breaking strength of at least 500 N.

2. Process according to claim 1, **characterised in that** the dried sub-portions in each case correspond to the mass of a unit of the dosage form.

3. Process according to claim 1 or 2, **characterised in that** before the addition of the solvent the formulation mixture is already dispersed in a liquid dispersant, in which the polymer component (C) is not soluble.

4. Process according to claim 3, **characterised in that** before or after the formulation composition is dispersed, it is already divided into sub-portions in each case corresponding to the mass of a unit of the dosage form.

5. Process according to claim 3 or 4, **characterised in that** the solvent and the dispersant are miscible with one another.

6. Process according to claim 1, **characterised in that** the solvent is incorporated into the formulation mixture as a foam.

7. Process according to claim 6, **characterised in that** the foam is stabilised with the assistance of foam stabilisers.

8. Process according to claim 6 or 7, **characterised in that** the composition granulated with the solvent foam is dried.

9. Process according to claim 8, **characterised in that** the dried, granulated composition is divided into sub-portions, which in each case correspond to the mass of a unit of the dosage form, and is shaped into the dosage form.

10. Process according to claim 1, **characterised in that** solvent is added to the formulation mixture in such an amount that a mouldable paste is obtained.

11. Process according to claim 10, **characterised in that** before or after it is dried, the paste is divided into sub-portions and the dried portions, optionally after being further divided in each case into a portion corresponding to the mass of a unit of the dosage form, are shaped or transformed into the dosage form.

12. Process according to claim 11, **characterised in that** the sub-portions have the form of strands.

13. Process according to claim 12, **characterised in that** the strands are produced with the assistance of a screen or a strand former.

14. Process according to claim 12, **characterised in that** the dried strands are singulated and shaped into the dosage form.

15. Process according to claim 14, **characterised in that** shaping proceeds with the assistance of a tablet press.

16. Process according to claim 12, **characterised in that** the dried strands are shaped into the dosage form with the assistance of shaping rollers or shaping belts equipped with rollers.

17. Process according to claim 11, **characterised in that** the paste is processed into a planar structure, out of which the dosage form is stamped.

18. Process according to claims 10 to 12, **characterised in that** the process is performed with the assistance of an extruder.

19. Process according to claim 1, **characterised in that** solvent is added to the formulation mixture in such a quantity that at least the polymer component (C) is dissolved.

20. Process according to claim 19, **characterised in that** the solution is processed into a planar structure.

21. Process according to claim 20, **characterised in that** the planar structure is obtained with the assistance of an extruder with a flat die or by casting the solution onto a level planar support.

22. Process according to claims 19 to 21, **characterised in that** the dosage form is shaped by stamping out of the dried planar structure or obtained by calendering.

23. Process according to claim 19, **characterised in that** the solution is divided into portions such that after drying they respectively correspond to the mass of a unit of the dosage form.

24. Process according to claim 23, **characterised in that** the portions are placed in moulds corresponding to the shape of a unit of the dosage form.

25. Process according to claim 19, **characterised in that** the solution is divided into any desired portions, which after drying are optionally recombined and shaped into the dosage form.

## Revendications

1. Procédé pour la préparation d'une forme d'administration solide présentant un potentiel d'abus au moins réduit, **caractérisé en ce que**
a) on ajoute, à un mélange de formulation contenant au moins une substance active présentant un potentiel d'abus (A), au moins un antioxydant comme adjuvant (B) et au moins un polymère synthétique ou naturel (C), qui présente une résistance à la rupture d'au moins 500 N, un solvant pour le polymère (C) au moins en des quantités telles que le mélange de formulation est humidifié régulièrement ;
b) le cas échéant, on répartit la masse ainsi au moins humidifiée en masses partielles ;
c) on sèche la ou les masse(s) ; et
d) on façonne en forme d'administration ;
le polymère (C) étant utilisé en des quantités telles que la forme d'administration présente également une résistance à la rupture d'au moins 500 N.

2. Procédé selon la revendication 1, **caractérisé en ce que** les masses partielles séchées correspondent à chaque fois à la masse d'une unité de la forme d'administration.

3. Procédé selon la revendication 1 ou 2, **caractérisé en ce que** le mélange de formulation est déjà dispersé, avant l'addition du solvant, dans un dispersant liquide dans lequel le composant polymère (C) n'est pas soluble.

4. Procédé selon la revendication 3, **caractérisé en ce que** la masse de formulation est déjà répartie avant ou après sa dispersion en masses partielles correspondant à chaque fois à la masse d'une unité de la forme d'administration.

5. Procédé selon la revendication 3 ou 4, **caractérisé en ce que** le solvant et le dispersant sont miscibles l'un avec l'autre.

6. Procédé selon la revendication 1, **caractérisé en ce que** le solvant est incorporé sous forme de mousse dans le mélange de formulation.

7. Procédé selon la revendication 6, **caractérisé en ce que** la mousse est stabilisée à l'aide de stabilisateurs de mousse.

8. Procédé selon la revendication 6 ou 7, **caractérisé en ce que** la masse granulée avec la mousse de solvant est séchée.

9. Procédé selon la revendication 8, **caractérisé en ce que** la masse séchée, granulée est répartie en masses partielles, qui correspondent à chaque fois à la masse d'une unité de la forme d'administration, et façonnée en forme d'administration.

10. Procédé selon la revendication 1, **caractérisé en ce qu'**on ajoute, au mélange de formulation, une quantité de solvant telle qu'on obtient une pâte pouvant être façonnée.

11. Procédé selon la revendication 10, **caractérisé en ce que** la pâte est répartie avant ou après son séchage en masses partielles et les masses séchées sont façonnées ou transformées en forme d'administration, le cas échéant après une autre répartition à chaque fois en une masse correspondant à la masse d'une unité de la forme d'administration.

12. Procédé selon la revendication 11, **caractérisé en ce que** les masses partielles présentent la forme de brins.

13. Procédé selon la revendication 12, **caractérisé en ce que** les brins sont produits à l'aide d'un tamis ou d'un dispositif de formation de brins.

14. Procédé selon la revendication 12, **caractérisé en ce que** les brins séchés sont séparés et façonnés en forme d'administration.

15. Procédé selon la revendication 14, **caractérisé en ce que** le façonnage s'effectue à l'aide d'une presse à comprimés.

16. Procédé selon la revendication 12, **caractérisé en ce que** les brins séchés sont façonnés en forme d'administration à l'aide de cylindres de façonnage ou de bandes de façonnage équipées de cylindres.

17. Procédé selon la revendication 11, **caractérisé en ce que** la pâte est transformée en une structure plane et séchée dont on découpe la forme d'administration.

18. Procédé selon les revendications 10 à 12, **caractérisé en ce que** le procédé est réalisé à l'aide d'une extrudeuse.

19. Procédé selon la revendication 1, **caractérisé en ce qu'**on ajoute, au mélange de formulation, une quantité de solvant telle qu'au moins le composant polymère (C) est dissous.

20. Procédé selon la revendication 19, **caractérisé en ce que** la solution est transformée en une structure plane.

21. Procédé selon la revendication 20, **caractérisé en ce que** la structure plane est obtenue à l'aide d'une extrudeuse avec une filière plate ou par déversement de la solution sur une sous-couche plane, plate.

22. Procédé selon la revendication 19 à 21, **caractérisé en ce que** la forme d'administration est façonnée par découpage ou obtenue par calandrage à partir de la structure plane, séchée.

23. Procédé selon la revendication 19, **caractérisé en ce que** la solution est répartie en des quantités partielles telles qu'elles correspondent après le séchage à chaque fois à la masse d'une unité de la forme d'administration.

24. Procédé selon la revendication 23, **caractérisé en ce que** les quantités partielles sont transvasées dans des moules correspondant à la forme d'une unité de la forme d'administration.

25. Procédé selon la revendication 19, **caractérisé en ce que** la solution est répartie en des quantités partielles quelconques, qui, après le séchage, le cas échéant sous forme à nouveau rassemblée, sont façonnées en forme d'administration.
